# EUROPEAN PATENT APPLICATION

(11) **EP 4 450 057 A1**
(43) Date of publication of application: **23.10.2024**
(21) Application number: 23169107.2
(22) Date of filing: 21.04.2023
(51) Int. Cl.: A61K 8/92, A61Q 19/00, A61Q 90/00

(54) **SKIN NOURISHING COMPOSITION**

(71) Applicant: Medela Holding AG, 6340 Baar (CH)
(72) Inventor: MIOTTO, Letizia, 8055 Zurich (CH); AMBROSECCHIA, Paola, 8044 Zurich (CH); OHIOZEUA, Jeremiah, 6330 Cham (CH); YANG, Wei, Toronto, M1B 5N4 (CA)
(74) Representative: Grünecker Patent- und Rechtsanwälte PartG mbB

(57) **Abstract**

The present invention relates to a composition comprising *Olea europaea* oil, *Persea gratissima* oil, *Euphorbia cerifera* cera and *Calendula officinalis* extract and to the use of such a composition for providing relief to sore nipples and dry skin.

## Description

### Field of the Invention

The present invention relates to a composition comprising *Olea europaea* oil, *Persea gratissima* oil, *Euphorbia cerifera* cera and *Calendula officinalis* extract. The present invention also relates to the use of such a composition for providing relief to sore nipples and dry skin.

### Background of the Invention

After giving birth, many breastfeeding women suffer from sore nipples and dry skin in the nipple region. This is due to increased sensitivity of the nipples after birth as well as the unaccustomed strain of suckling and can be a source of great discomfort. Sore nipples are in fact a very common reason for premature weaning. It is estimated that about one third of mothers report having weaned earlier than they would like due to sore nipples.

Over the centuries, breastfeeding women have tried various means to relieve sore nipples. However, many traditional measures provide only limited relief and may even exacerbate complaints by further drying the skin in the nipple region. Current attempts to alleviate nipple soreness and dry skin, in particular dry skin in the area of the nipples, of breastfeeding women, aim to preserve the moisture of the skin. No one means of alleviating nipple soreness and/or dry skin in the nipple region has yet established itself as dominant.

In view of the above there still remains a need for products which can be easily applied and provide relief to sore nipples and/or dry skin.

### Summary of the Invention

Unexpectedly, it has been found that a composition comprising *Olea europaea* oil, *Persea gratissima* oil, *Euphorbia cerifera* cera and *Calendula officinalis* extract provides benefits such as naturally relieving dry skin and/or sore nipples of breastfeeding women.

In a first aspect, the present invention relates to a composition comprising *Olea europaea* oil, *Persea gratissima* oil, *Euphorbia cerifera* cera and *Calendula officinalis* extract.

In a further aspect, the present invention relates to the cosmetic use of the composition in relieving sore nipples and/or dry skin.

### Detailed description of the invention

In one aspect, the present invention relates to a composition comprising *Olea europaea* oil, *Persea gratissima* oil, *Euphorbia cerifera* cera and *Calendula officinalis* extract.

As will be discussed in greater detail below, the composition according to the invention provides a number of unique advantages in naturally relieving dry skin and, in particular, sore nipples when used by breast-feeding or breast milk-pumping women. The inventors have found that the ingredients specified above and explained in greater detail below combine the advantages of a composition with lightweight viscosity for easy applicability to sore areas of the body, e.g. nipples and nipple regions, a natural origin which obviates concerns of ingestion and which allows use as a "leave-on product" which need not be removed between breast-feeding or breast milk-pumping sessions, while conditioning and soothing the area to which the inventive composition is applied. Further embodiments and advantages of the invention are discussed below.

In a preferred embodiment in optional combination with any of the above or below aspects or embodiments, the *Olea europaea* oil is *Olea europaea* fruit oil (such as for example designated by CAS (Chemical Abstracts Services) registry number 8001-25-0), and/or the *Calendula officinalis* extract is *Calendula officinalis* flower extract (such as for example designated by CAS registry number 84776-23-8). In a further preferred embodiment in optional combination with any of the above or below aspects or embodiments, the *Olea europaea* oil is *Olea europaea* fruit oil, and the *Calendula officinalis* extract is *Calendula officinalis* flower extract.

The *Persea gratissima* oil herein may for example be designated by CAS registry number 8024-32-6).

The *Euphorbia cerifera* cera herein may for example be designated by CAS registry number 8006-44-8.

In an embodiment in optional combination with any of the above or below aspects or embodiments, the composition comprises about 30% to about 90% by weight, preferably about 40% to about 80% by weight, more preferably about 50% to about 70% by weight, even more preferably about 58% to about 60% by weight, most preferably about 59% by weight of the *Olea europaea* oil.

In an embodiment in optional combination with any of the above or below aspects or embodiments, the composition comprises about 13% to about 53% by weight, preferably about 20% to about 46% by weight, more preferably about 27% to about 39% by weight, even more preferably about 32% to about 35% by weight, most preferably about 33.5% by weight of the *Persea gratissima* oil.

In an embodiment in optional combination with any of the above or below aspects or embodiments, the composition comprises about 1% to about 8% by weight, preferably about 2% to about 7% by weight, more preferably about 3% to about 6% by weight, even more preferably about 4% to about 5% by weight, most preferably about 4.5% by weight of the *Euphorbia cerifera* cera.

In an embodiment in optional combination with any of the above or below aspects or embodiments, the composition comprises about 0.1 % to about 7% by weight, preferably about 0.5% to about 5.5% by weight, more preferably about 1.5% to about 4.5% by weight, even more preferably 2.5% to about 3.5% by weight, most preferably about 3% by weight of the *Calendula officinalis* extract.

In an embodiment in optional combination with any of the above or below aspects or embodiments, the cumulative amount of *Olea europaea* oil and *Persea gratissima* oil in the composition is about 80% to about 98% by weight, preferably about 85% to about 97% by weight, more preferably about 90% to about 95% by weight, most preferably about 92.5% by weight.

In an embodiment in optional combination with any of the above or below aspects or embodiments, the cumulative amount of the *Olea europaea* oil, *Persea gratissima* oil, *Euphorbia cerifera* cera and *Calendula officinalis* extract in the composition is more than about 90% by weight, preferably more than about 95% by weight, more preferably more than about 98% by weight.

In a preferred embodiment in optional combination with any of the above or below aspects or embodiments, the composition essentially consists of *Olea europaea* oil, *Persea gratissima* oil, *Euphorbia cerifera* cera and *Calendula officinalis* extract.

In an embodiment in optional combination with any of the above or below aspects or embodiments, the ingredients of the composition according to the invention are of certified organic origin, for example Cosmos (COSMetic Organic and natural Standard) certification, e.g. Ecocert certification.

In an embodiment in optional combination with any of the above or below aspects or embodiments, the composition is in the form of a balm, a cream, a lotion, an ointment, a gel, a milk or a salve.

In a further aspect, the present invention relates to a cosmetic use of the above composition for relieving sore nipples and/or dry skin.

Technical and scientific terms used hereafter carry the commonly understood meaning of the word unless defined otherwise.

It is to be understood that section headings as used throughout the present application are merely for organizational purposes, and do not confer exclusive character to the disclosure pertaining to aspects of the invention mentioned in a section headings. Section headings thus do not exclude the combination of the disclosure within any one section heading to a corresponding or analogous context provided under another section heading. Such intra-section disclosures are explicitly within the disclosure of the present application.

As used herein, each of the terms "comprising', "having" and "containing", including grammatical variants thereof, are meant in a non-exhaustive sense to mean "including", but not necessarily "composed of", and does not exclude elements in addition to those explicitly recited as being present. As used herein, then, the terms "comprising", "having" and "containing", and grammatical variants thereof, indicate that components other than those explicitly recited may, but need not, be present. As such these terms include as a limiting case embodiments in which no other elements than those recited are present, e.g. in the commonly accepted sense of "consisting of'.

As used herein, the phrase "consisting of", and grammatically related variants thereof, means that no other elements are present than those recited. In standing with the above definition of "comprising" (and grammatically and semantically related terms), the term "consisting of" therefore denotes a limiting scenario within the meaning of "comprising".

Unless defined otherwise, any feature within any aspect or embodiment of the invention may be combined with any feature within any other aspect or embodiment of the invention, and the skilled person understands such combination as being encompassed in the original disclosure of the present application. This applies in particular to all embodiments described within the section relating to the composition *per se,* in respect of other aspects, e.g. methods and uses, of those compositions. This also applies in particular, but not exclusively, to endpoints of ranges disclosed herein. For instance, if a given substance is disclosed as existing in a composition in a concentration range of about X-Y% or about A-B%, the present application is to be understood as explicitly disclosing not only the ranges about X-Y% and about A-B%, but also the ranges about X-B%, about A-Y% and, in as far as numerically possible, about Y-A% and about B-X%. Each of these ranges, and range combinations, are contemplated, and are to be understood as being directly and unambiguously disclosed in the present application. Unless stated otherwise, the designation of a range in the present application using a hyphen ("-") separating two bracketing values X and Y, or two bracketing ratios, is to be understood as meaning and disclosing the specified range in which both endpoint values X and Y are included. The same applies to a range expressed as "from about X to about Y". Accordingly, the expressions of ranges as "X -Y", "of X to Y", "from X to Y", "of X - Y" and "from X - Y" are to be understood equivalently as meaning and disclosing a range encompassing the end value X, all values between X and Y, as well as the end value Y.

The designation of a range in the present application using the word "between" preceding two bracketing values X and Y, or two bracketing ratios, is to be understood as meaning and disclosing the specified range in which both endpoint values X and Y are excluded, but all values between the specified endpoint values X and Y are included. As used herein the term "about" when referring to a particular value, e.g. an endpoint or endpoints of a range, encompasses and discloses, in addition to the specifically recited value itself, a certain variation around the specifically recited value. Such a variation may for example arise from normal measurement variability, e.g. in the weighing or apportioning of various substances by methods known to the skilled person. The term "about" shall be understood as encompassing and disclosing a range of variability above and below an indicated specific value, said percentage values being relative to the specific recited value itself, as follows. The term "about" may denote variability of ± 5.0%. The term "about" may denote variability of ± 4.9%. The term "about" may denote variability of ± 4.8%. The term "about" may denote variability of ± 4.7%. The term "about" may denote variability of ± 4.6%. The term "about" may denote variability of ± 4.5%. The term "about" may denote variability of ± 4.4%. The term "about" may denote variability of ± 4.3%. The term "about" may denote variability of ± 4.2%. The term "about" may denote variability of ± 4.1%. The term "about" may denote variability of ± 4.0%. The term "about" may denote variability of ± 3.9%. The term "about" may denote variability of ± 3.8%. The term "about" may denote variability of ± 3.7%. The term "about" may denote variability of ± 3.6%. The term "about" may denote variability of ± 3.5%. The term "about" may denote variability of ± 3.4%. The term "about" may denote variability of ± 3.3%. The term "about" may denote variability of ± 3.2%. The term "about" may denote variability of ± 3.1%. The term "about" may denote variability of ± 3.0%. The term "about" may denote variability of ± 2.9%. The term "about" may denote variability of ± 2.8%. The term "about" may denote variability of ± 2.7%. The term "about" may denote variability of ± 2.6%. The term "about" may denote variability of ± 2.5%. The term "about" may denote variability of ± 2.4%. The term "about" may denote variability of ± 2.3%. The term "about" may denote variability of ± 2.2%. The term "about" may denote variability of ± 2.1%. The term "about" may denote variability of ± 2.0%. The term "about" may denote variability of ± 1.9%. The term "about" may denote variability of ± 1.8%. The term "about" may denote variability of ± 1.7%. The term "about" may denote variability of ± 1.6%. The term "about" may denote variability of ± 1.5%. The term "about" may denote variability of ± 1.4%. The term "about" may denote variability of ± 1.3%. The term "about" may denote variability of ± 1.2%. The term "about" may denote variability of ± 1.1%. The term "about" may denote variability of ± 1.0%. The term "about" may denote variability of ± 0.9%. The term "about" may denote variability of ± 0.8%. The term "about" may denote variability of ± 0.7%. The term "about" may denote variability of ± 0.6%. The term "about" may denote variability of ± 0.5%. The term "about" may denote variability of ± 0.4%. The term "about" may denote variability of ± 0.3%. The term "about" may denote variability of ± 0.2%. The term "about" may denote variability of 0.1%. The term "about", in reference to the particular recited value, may denote that exact particular value itself, irrespective of any explicit mention that this exact particular value is included; even in the absence of an explicit indication that the term "about" includes the particular exact recited value, this exact particular value is still included in the range of variation created by the term "about", and is therefore disclosed. Unless stated otherwise, if the term "about" is recited before the first endpoint of a numerical range, this term refers to both the first endpoint of the range and the second endpoint of the range. For instance, a recited range of "about X to Y" should be read as "about X to about Y".

In the present application, amounts are often provided in "% by weight". It is to be understood herein that "% by weight" refers to the weight percent of a particular ingredient or ingredients in respect of the total weight of the composition.

As the skilled person will know, a trivial name for *Olea europea* is "olive", a trivial name for *Persea gratissima* is "avocado", a trivial name for *Euphorbia cerifera* is "candelilla" and a trivial name for *Calendula offinalis* is "marigold".

The present invention relates to a composition comprising *Olea europaea* oil, *Persea gratissima* oil, *Euphorbia cerifera* cera and *Calendula officinalis* extract.

In a preferred embodiment in optional combination with any of the above or below aspects or embodiments, the *Olea europaea* oil is *Olea europaea* fruit oil and/or the *Calendula officinalis* extract is *Calendula officinalis* flower extract. In a further preferred embodiment in optional combination with any of the above or below aspects or embodiments, the *Olea europaea* oil is *Olea europaea* fruit oil, and the *Calendula officinalis* extract is *Calendula officinalis* flower extract.

In a preferred embodiment in optional combination with any of the above or below aspects or embodiments, the composition essentially consists of *Olea europaea* fruit oil, *Persea gratissima* oil, *Euphorbia cerifera* cera and *Calendula officinalis* flower extract.

In the context of the present invention "essentially consisting of" is meant to exclude the presence of other ingredients except possibly in trace amounts, e.g. amounts near the limit of detection for such ingredients. In its furthest limit, in a preferred embodiment, this term is to be interpreted as "consisting entirely of", that is only the recited ingredients are present and nothing else.

As used herein, a substance which is "solid" means that the substance can substantially sustain its physical shape when unsupported by external means, and retains this characteristic at room temperature and up to a temperature of at least 45°C.

The mixture of (fruit) oils and calendula (flower) extract confers a naturally pleasant scent on the composition. Thus, the composition can ideally be formulated without any natural or synthetic fragrances, in particular without any natural or synthetic fragrances typically used in cosmetic products such as e.g. linalool or geraniol. In a preferred embodiment, the present composition is formulated without any natural or synthetic fragrances.

Butylated hydroxytoluene (BHT), also known as dibutylhydroxytoluene, is a lipophilic organic compound with antioxidant properties. Due to these properties, BHT is sometimes added to numerous consumer products in the food and cosmetic industry to prevent or slow down changes of products by atmospheric oxygen. However, like many closely related phenol antioxidants, BHT has a low, albeit acute toxicity. In a preferred embodiment, the present composition is formulated without BHT.

*Euphorbia cerifera* cera, a natural wax, is known as an effective binding agent that combines well with oils such as *Olea europaea* (fruit) oil and *Persea gratissima* oil. Solvents such as water or diluents are not required in the present composition. In a preferred embodiment, the present composition is therefore formulated without water. In such a preferred embodiment, the composition exists as a single oily phase, an aqueous phase being absent. Consequently, the addition of surfactants is not required and the present composition is also preferably formulated without a surfactant, e.g. a polyoxyalkylene carboxylate surfactant.

In an embodiment in optional combination with any of the above or below aspects or embodiments, the composition of the present invention is formulated without additives such as e.g. preservatives, fragrances, emulsifying agents or surfactants.

In a preferred embodiment in optional combination with any of the above or below aspects or embodiments, the inventive composition is formulated without a vitamin such as vitamin B, vitamin D or a tocopherol (e.g. vitamin E or tocopherol acetate), or vitamin A.

In a preferred embodiment in optional combination with any of the above or below aspects or embodiments, the inventive composition is formulated without a propellant, e.g. a hydrocarbon propellant, a gas propellant, in particular a liquefied gas propellant.

In a preferred embodiment in optional combination with any of the above or below aspects or embodiments, the inventive composition is formulated without a foaming agent.

In a preferred embodiment in optional combination with any of the above or below aspects or embodiments, the inventive composition is formulated without palm oil, coconut oil, primrose oil, flax oil, or pumpkin oil.

In a preferred embodiment in optional combination with any of the above or below aspects or embodiments, the inventive composition is formulated without a paraben.

In a preferred embodiment in optional combination with any of the above or below aspects or embodiments, the inventive composition is formulated without alcohol, e.g. cetyl alcohol, stearyl alcohol, a C₁-C₆ alcohol.

In a preferred embodiment in optional combination with any of the above or below aspects or embodiments, the inventive composition is formulated without an antibiotic, in particular a minocycline antibiotic or a tetracycline antibiotic.

In a preferred embodiment in optional combination with any of the above or below aspects or embodiments, the inventive composition includes only ingredients of vegetable origin. That is, the inventive composition is formulated without any ingredient of animal origin, e.g. lanolin, a birdfeather oil, propolis or beeswax.

In a preferred embodiment in optional combination with any of the above or below aspects or embodiments, the inventive composition is formulated without any solid substance, e.g. a solid particle, a nanoparticle, an absorbent material, a fabric, a clay (e.g. a bentonite such as e.g. quaternium-18 bentonite, a hectorite or organophilic clay (e.g. stearalkonium hectorite, quaternium-18 hectorite), a silica (e.g. magnesium aluminum silicate) or a cloth.

In a preferred embodiment in optional combination with any of the above or below aspects or embodiments, the inventive composition is formulated without any inclusion of gas, e.g a foam.

In a preferred embodiment in optional combination with any of the above or below aspects or embodiments, the inventive composition is formulated without any flower wax of jasmine, lavender, rose, raspberry or violet.

In a preferred embodiment in optional combination with any of the above or below aspects or embodiments, the inventive composition is formulated without (hydrogenated) jojoba (seed) oil, meadowfoam (seed) oil, fennel (seed) oil, flax (seed) oil, almond oil, sesame oil, vine (fruit) oil, grape (seed) oil, oregano oil, apricot oil, fish oil, geranium oil, ylang ylang oil, soy oil, soy isoflavone-3, oyster oil, linseed oil, white leaf oil, safflower (seed) oil, sunflower (seed) oil, macadamia nut oil, castor seed oil, peony seed oil, grapefruit seed oil, apple seed oil, pomegranate seed oil, watermelon seed oil, Cucurbita pepo seed oil, rapeseed oil, sesame seed oil, white chia seed oil, zucchini seed oil, walnut seed oil, baobab seed oil, bitter oil seed oil, *Borago Officinalis* seed oil, *Sclerocarya birrea* seed oil, cotton seed oil, tea seed oil, COIX LACRYMA-JOBI MA-YUEN seed oil, papaya seed oil, raspberry seed oil, monkey bread tree seed oil, bitter oil tree seed oil, moringa seed oil, American pecan seed oil, perilla seed oil, Babassu seed oil, white lupin seed oil, vanillin, lavender oil, Melaleuca alterniflora oil, cephalaria oil, chamomile oil, neem oil, citrus oil, orange oil, frankincense oil, cannabis (seed) oil, or sunflower (seed) wax.

In a preferred embodiment in optional combination with any of the above or below aspects or embodiments, the inventive composition is formulated without a fruit extract (e.g. mango fruit extract), coconut fruit extract, dandelion (root) extract, Matricaria leaf extract, Glycyrrhiza glabra root extract, Centella asiatica extract, Rosemary leaf extract, Scutellaria root extract, tea extract, Polygonum cuspidatum root extract, swertia chirata extract, turmeric, orange extract, frankincense extract, cannabis (seed) extract, or rose flower extract.

In a preferred embodiment in optional combination with any of the above or below aspects or embodiments, the inventive composition is formulated without bis-abolol, calamine or zinc oxide.

In a preferred embodiment in optional combination with any of the above or below aspects or embodiments, the inventive composition is formulated without a solid phase.

In a preferred embodiment in optional combination with any of the above or below aspects or embodiments, the inventive composition is formulated without essential oils, in particular 90 or more essential oils.

In a preferred embodiment in optional combination with any of the above or below aspects or embodiments, the inventive composition is formulated without a surfactant, in particular an anionic surfactant, a nonionic surfactant, a zwitterionic surfactant, an amphoteric surfactant, a biosurfactant (e.g. a monoramnolipid, a dirramnolipid, a soforolipid, a mannosileritritol lipid, a celubiose, a xylolipid, a trehalipid, a lipopeptide, a glycoside or a rhamnose), a polyoxyalkylene carboxylate surfactant, or a tenside.

In a preferred embodiment in optional combination with any of the above or below aspects or embodiments, the inventive composition is formulated without (hydrogenated) castor oil.

In a preferred embodiment in optional combination with any of the above or below aspects or embodiments, the inventive composition is formulated without petrolatum or a mineral oil.

In a preferred embodiment in optional combination with any of the above or below aspects or embodiments, the inventive composition is formulated without rice bran wax.

In a preferred embodiment in optional combination with any of the above or below aspects or embodiments, the inventive composition is formulated without (hydrogenated) phosphatidylcholine.

In a preferred embodiment in optional combination with any of the above or below aspects or embodiments, the inventive composition is formulated without cyclopentasiloxane, polyethylene glycol (PEG), polypropylene glycol (PPG), PEG/PPG, in particular PEG/PPG 18/18, or dimethicone.

In a preferred embodiment in optional combination with any of the above or below aspects or embodiments, the inventive composition is formulated without cinnamon bark (extract).

In a preferred embodiment in optional combination with any of the above or below aspects or embodiments, the inventive composition is formulated without tea residues.

In a preferred embodiment in optional combination with any of the above or below aspects or embodiments, the inventive composition is formulated without polyvinyl alcohol (PVA).

In a preferred embodiment in optional combination with any of the above or below aspects or embodiments, the inventive composition is formulated without any saponin, e.g. a saponin of *Sapindus mukorossi.*

In a preferred embodiment in optional combination with any of the above or below aspects or embodiments, the inventive composition is formulated without a substance, in particular an oil, which has been processed in such a way to convey one or more aromatic substances to the produced substance, in particular to the produced oil.

In a preferred embodiment in optional combination with any of the above or below aspects or embodiments, the inventive composition is formulated without a stearate, (e.g. glycerol stearate, PEG-stearate, (e.g. PEG-100 stearate), sucrose distearate), PEG-7 olive oil carboxylate, or glyceryl oleate.

In a preferred embodiment in optional combination with any of the above or below aspects or embodiments, the inventive composition is formulated without propanediol, glycerin (glycerine, glycerol), a triglyceride (e.g. a caprylic or capric triglyceride), a biosaccharide, dimethyl isosorbide, ethyoxy diglycerol, or a polycorbate (e.g. polysorbate 20).

In a preferred embodiment in optional combination with any of the above or below aspects or embodiments, the inventive composition is formulated without a moisturizer, e.g. a humectant.

In a preferred embodiment in optional combination with any of the above or below aspects or embodiments, the inventive composition is formulated without 25-40 wt% vegetable wax.

In a preferred embodiment in optional combination with any of the above or below aspects or embodiments, the inventive composition is formulated without a polyphenol or 2-methyloxolane.

In a preferred embodiment in optional combination with any of the above or below aspects or embodiments, the inventive composition is formulated without olive leaf extract.

In a preferred embodiment in optional combination with any of the above or below aspects or embodiments, the inventive composition is formulated without a lignin.

In a preferred embodiment in optional combination with any of the above or below aspects or embodiments, the inventive composition is formulated without an alkyl benzoate (e.g. an C₁₂-C₁₅ alkyl benzoate), isopropyl myristrate, isopropyl palmitate, octyldodecanol, decyl oleate, a cocoglyceride, ethylhexyl stearate, ceteraryl isononanoate, cetearyl ethyhexanonate, decyl cocoate, cetyl dimethicone, ethylhexyl palmitate, PPG-11 stearyl ether, PPG-15 stearyl ether, or PPG-14 butyl ether.

In a preferred embodiment in optional combination with any of the above or below aspects or embodiments, the inventive composition is formulated without ginger extract.

In a preferred embodiment in optional combination with any of the above or below aspects or embodiments, the inventive composition is formulated without a hydrocarbon, a sterol, a tocotrienol, a carotenoid pigment or a xanthophyll.

In a preferred embodiment in optional combination with any of the above or below aspects or embodiments, the inventive composition is formulated without an acid, e.g. a fruit acid, a linoleic acid (e.g. alpha-linoleic acid, gamma-linoleic acid), docosahexanioc acid, eicosapentanoic acid, an omega fatty acid (e.g. an omega-3 fatty acid or an omega-6 fatty acid), a sialic acid or a benzoic acid (e.g. hydroxyphenyl propionamide benzoic acid).

In a preferred embodiment in optional combination with any of the above or below aspects or embodiments, the inventive composition is formulated without a dye or colorant. In a preferred embodiment in optional combination with any of the above or below aspects or embodiments, the inventive composition is formulated without an antioxidant.

In a preferred embodiment in optional combination with any of the above or below aspects or embodiments, the inventive composition is formulated without a panthenol (e.g. D-panthenol), a lauric acid (e.g. lauric acid monoglyceride), hyaluronic acid or a salt thereof such as for example sodium hyaluronate, a phospholipid.

In a preferred embodiment in optional combination with any of the above or below aspects or embodiments, the inventive composition is formulated without a sterol, e.g. a phytosterol.

In a preferred embodiment in optional combination with any of the above or below aspects or embodiments, the inventive composition is formulated without an emulsifier, e.g. a lecithin.

In a preferred embodiment in optional combination with any of the above or below aspects or embodiments, the inventive composition is formulated without a skin exfoliative or a skin polishing material.

In a preferred embodiment in optional combination with any of the above or below aspects or embodiments, the inventive composition is formulated without a solvent.

In a preferred embodiment in optional combination with any of the above or below aspects or embodiments, the inventive composition is formulated without a peroxide.

In a preferred embodiment in optional combination with any of the above or below aspects or embodiments, the inventive composition is formulated without an alkyl ketal ester, methyl levulinate propylene glycol ketal, or ethyl levulinate propylene glycol ketyl.

In a preferred embodiment in optional combination with any of the above or below aspects or embodiments, the inventive composition is formulated without vegetable butter. In a preferred embodiment in optional combination with any of the above or below aspects or embodiments, the inventive composition is formulated without shea butter.

In a preferred embodiment in optional combination with any of the above or below aspects or embodiments, the inventive composition is formulated without squalene.

In a preferred embodiment in optional combination with any of the above or below aspects or embodiments, the inventive composition is formulated without glycereth-26.

In a preferred embodiment in optional combination with any of the above or below aspects or embodiments, the inventive composition is formulated without bis-albolol.

In a preferred embodiment in optional combination with any of the above or below aspects or embodiments, the inventive composition is formulated without *Calophyllum inophyllum, Citrus aurantium, Eucalyptus globulus, Eugenia caryophyllata, Foeniculum vulgare, Helichrysum angustifolia, Juniperus virginiana, Lavendula officinalis, Myristica fragrans, Ocimum basilicum, Pinus sylvestris, Piper nigrum, Rosemarinus officinalis, Salvia officinalis lamiacae* or *Salvia sclarea.*

In a preferred embodiment in optional combination with any of the above or below aspects or embodiments, the inventive composition is formulated without Allium spp. extract, Aloe spp. extract, Cinnamomum spp. extract, Curcuma spp. extract, Lavandula spp. extract, Melaleuca spp. extract, Plantago extract, Salvia officinalis extract, Salvia miltiorrhiza extract, or Thymus spp. extract,

In a preferred embodiment in optional combination with any of the above or below aspects or embodiments, the inventive composition is formulated without a ketal.

In a preferred embodiment in optional combination with any of the above or below aspects or embodiments, the inventive composition is formulated without a cyclodextrin.

In a preferred embodiment in optional combination with any of the above or below aspects or embodiments, the inventive composition is formulated without sodium acrylate, sodium acryloyl dimethyl taurate, or a sodium acrylate/sodium acryloyl dimethyl taurate copolymer.

In addition, the ingredients in the inventive composition are advantageously - and preferably - non-toxic and certified organic. The natural, organic nature of the inventive composition has the effect that the breast-feeding mother need not be concerned about possible ingestion of the inventive composition by the infant. Following application of the inventive composition to sore and/or dry nipples after a breast-feeding or pumping session, the inventive composition can therefore be left on the nipples, i.e. does not need to be washed off, prior to subsequent breast-feeding or pumping sessions to prevent unwanted ingestion by the breast-feeding infant, or by the infant who will ingest pumped breast milk. The inventive composition is thus advantageously a "leave-on product".

This is advantageous for a number of reasons. For instance, the ability to leave the inventive composition on dry and/or sore nipples means that the breast-feeding or breast milk-pumping mother need not plan in time to wash off a previous application of the inventive composition prior to any following breast-feeding or -pumping session. The breast-feeding or breast milk-pumping mother thus gains additional time to accrue the benefit of relief from a previous application of the inventive composition, while also maximizing time between breast-feeding or pumping sessions for other activities such as e.g. regenerative sleep, which is an especially precious commodity in the daily routine of any new mother. Further, the ability to dispense with intermittent washing cycles also means that the breast-feeding or breast milk-pumping mother can avoid further insulting already-sore nipples by the irritating mechanical process of washing and drying.

Of course, the inclusion of additives need not automatically disqualify such a composition from classification as a "leave-on product", but the lack of any need to consider and additionally insure the safety and e.g. organic certification of any such additives or preservatives constitutes a particular advantage of the composition of the present application.

Lacking any additives such as e.g. preservatives, fragrances or surfactants, the composition of the present invention has the additional advantage of being hypoallergenic and can thus be used by adults and children, including babies, to relieve sore or dry skin on any part of the body. In an embodiment of the present invention, it is therefore contemplated that the inventive composition can be used cosmetically by subjects having normal to dry and/or sore skin anywhere on the body. In standing with the above, however, the composition of the present invention is particularly useful for breastfeeding women who often suffer from sore nipples and/or dry skin in the nipple region, and as mentioned above is particularly well-suited for direct application to sore and/or dry nipples and the surrounding nipple region to relieve such soreness and dryness between breastfeeding sessions. As mentioned above, the non-toxic nature of the inventive composition, and in particular its organic certification, has the advantage that the composition can be safely used as a leave-on product for the relief of sore nipples and dry skin, with the associated advantages described above.

In a preferred embodiment in optional combination with any of the above or below aspects or embodiments, the cumulative amount of *Olea europaea* (fruit) oil and *Persea gratissima* oil in the composition is about 80% to about 98% by weight, such as about 81% to about 98%, about 82% to about 98%, about 83% to about 98%, about 84% to about 98%, about 85% to about 98%, about 86% to about 98%, about 87% to about 98%, about 88% to about 98%, about 89% to about 98%, about 90% to about 98%, about 91% to about 98%, about 92% to about 98%, about 93% to about 98%, about 94% to about 98%, about 80% to about 97%, about 80% to about 96%, about 80% to about 95%, about 81% to about 97%, about 82% to about 97%, about 82% to about 96%, about 82% to about 95%, about 83% to about 97%, about 83% to about 96%, about 83% to about 95%, about 84% to about 97%, about 84% to about 96% or about 84% to about 95% by weight.

More preferably, also in optional combination with any of the above or below aspects or embodiments, the cumulative amount of *Olea europaea* (fruit) oil and *Persea gratissima* oil in the composition is about 85% to about 97% by weight, such as about 86 to about 97%, about 86 to about 96%, about 86 to about 95%, about 87 to about 97%, about 87 to about 96%, about 87 to about 95%, about 88 to about 97%, about 88 to about 96%, about 88 to about 95%, about 89 to about 97%, about 89 to about 96% or about 89 to about 95% by weight.

In a more preferred embodiment in optional combination with any of the above or below aspects or embodiments, the cumulative amount of *Olea europaea* (fruit) oil and *Persea gratissima* oil in the composition is about 90% to about 95% by weight, such as about 91 to about 95%, about 92 to about 95%, about 93 to about 95% or about 94 to about 95% by weight.

It is especially preferred, also in optional combination with any of the above or below aspects or embodiments, that the cumulative amount of *Olea europaea* (fruit) oil and *Persea gratissima* oil in the composition is about 92.5% by weight.

In an embodiment in optional combination with any of the above or below aspects or embodiments, *Olea europaea* (fruit) oil is present in the composition in an amount of about 30% to about 90% by weight, such as about 30% to about 88%, about 30% to about 86%, about 30% to about 84%, about 30% to about 82%, about 32% to about 88%, about 32% to about 86%, about 32% to about 84%, about 32% to about 82%, about 34% to about 88%, about 34% to about 86%, about 34% to about 84%, about 34% to about 82%, about 36% to about 88%, about 36% to about 86%, about 36% to about 84%, about 36% to about 82%, about 38% to about 88%, about 38% to about 86%, about 38% to about 84%, or about 38% to about 82% by weight.

In a preferred embodiment in optional combination with any of the above or below aspects or embodiments, *Olea europaea* (fruit) oil is present within the composition in an amount of about 40% to about 80% by weight, such as about 40% to about 78%, about 40% to about 76%, about 40% to about 74%, about 40% to about 72%, about 42% to about 78%, about 42% to about 76%, about 42% to about 74%, about 42% to about 72%, about 44% to about 78%, about 44% to about 76%, about 44% to about 74%, about 44% to about 72%, about 46% to about 78%, about 46% to about 76%, about 46% to about 74%, about 46% to about 72%, about 48% to about 78%, about 48% to about 76%, about 48% to about 74%, or about 48% to about 72% by weight.

In a more preferred embodiment in optional combination with any of the above or below aspects or embodiments, *Olea europaea* (fruit) oil is present in the composition in an amount of about 50% to about 70% by weight, such as about 50% to about 68%, about 50% to about 66%, about 50% to about 64%, about 50% to about 62%, about 52% to about 68%, about 52% to about 66%, about 52% to about 64%, about 52% to about 62%, about 54% to about 68%, about 54% to about 66%, about 54% to about 64%, about 54% to about 62%, about 56% to about 68%, about 56% to about 66%, about 56% to about 64%, about 56% to about 62%, about 58% to about 68%, about 58% to about 66%, about 58% to about 64%, or about 58% to about 62% by weight.

Even more preferably in optional combination with any of the above or below aspects or embodiments, *Olea europaea* (fruit) oil is present in the composition in an amount of about 58% to about 60% by weight. Most preferably, also in combination with any of the above or below embodiments, Olea *europaea* (fruit) oil is present in the composition in an amount of about 59% by weight.

In an embodiment in optional combination with any of the above or below aspects or embodiments, *Persea gratissima* oil is present in the composition in an amount of about 13% to about 53% by weight, such as about 13% to about 51%, about 13% to about 49%, about 13% to about 47%, about 15% to about 53%, about 14% to about 51%, about 14% to about 49%, about 15% to about 47%, about 17% to about 53%, about 17% to about 51%, about 17% to about 49%, about 17% to about 47%, about 19% to about 53%, about 19% to about 51%, about 19% to about 49%, or about 19% to about 47% by weight.

In preferred embodiment in optional combination with any of the above or below aspects or embodiments, *Persea gratissima* oil is present in the composition in an amount of about 20% to about 46% by weight, such as about 20% to about 44%, about 20% to about 42%, about 20% to about 40%, about 22% to about 46%, about 22% to about 44%, about 22% to about 42%, about 22% to about 40%, about 24% to about 46%, about 24% to about 44%, about 24% to about 42%, about 24% to about 40%, about 26% to about 46%, about 26% to about 44%, about 26% to about 42%, or about 26% to about 40% by weight.

In a more preferred embodiment in optional combination with any of the above or below aspects or embodiments, *Persea gratissima* oil is present in the composition in an amount of about 27% to about 39% by weight, such as about 27% to about 38%, about 27% to about 37%, about 27% to about 36%, about 28% to about 39%, about 28% to about 38%, about 28% to about 37%, about 28% to about 36%, about 29% to about 39%, about 29% to about 38%, about 29% to about 37%, about 29% to about 36%, about 30% to about 39%, about 30% to about 38%, about 30% to about 37%, about 30% to about 36%, about 31% to about 39%, about 31% to about 38%, about 31% to about 37%, or about 31% to about 36% by weight.

Even more preferably, again in optional combination with any of the above or below aspects or embodiments, *Persea gratissima* oil is present in the composition in an amount of about 32% to about 35% by weight, such as about 32% to about 34% or about 31% to about 35% by weight. Most preferably, in optional combination with any of the above or below aspects or embodiments, *Persea gratissima* oil is present in the composition in an amount of about 33.5% by weight.

In an embodiment in optional combination with any of the above or below aspects or embodiments, *Euphorbia cerifera* cera is present in the composition in an amount of about 1% to about 8% by weight, such as about 1% to about 7%, about 1% to about 6.5%, about 2% to about 8%, about 2% to about 7%, about 2% to about 6.5%, about 2.5% to about 8%, about 2.5% to about 7%, or about 2.5% to about 6.5% by weight.

In preferred embodiment in optional combination with any of the above or below aspects or embodiments, *Euphorbia cerifera* cera is present in the composition in an amount of about 2% to about 7% by weight, such as about 3% to about 7%, or about 2% to 6% by weight.

In a more preferred embodiment in optional combination with any of the above or below aspects or embodiments, *Euphorbia cerifera* cera is present in the composition in an amount of about 3% to about 6% by weight, such as about 3% to about 5.5%, about 3.5% to about 6%, or about 3.5% to about 5.5% by weight, even more preferably about 4% to about 5% by weight. Most preferably in optional combination with any of the above or below aspects or embodiments, *Euphorbia cerifera* cera is present in the composition in an amount of about 4.5% by weight.

In an embodiment in optional combination with any of the above or below aspects or embodiments, *Calendula officinalis* (flower) extract is present in the composition in an amount of about 0.1% to about 7% by weight, such as about 0.1% to about 6.5%, about 0.1% to about 6%, about 0.2% to about 7% by weight, about 0.2% to about 6.5%, about 0.2% to about 6%, about 0.3% to about 7% by weight, about 0.3% to about 6.5%, about 0.3% to about 6%, about 0.4% to about 7% by weight, about 0.4% to about 6.5%, or about 0.4% to about 6% by weight.

In preferred embodiment in optional combination with any of the above or below aspects or embodiments, *Calendula officinalis* (flower) extract is present in the composition in an amount of about 0.5% to about 5.5% by weight, such as about 0.6% to about 5.0% by weight, about 0.7% to about 5.5% by weight, about 0.7% to about 5.0%, about 0.8% to about 5.5% by weight, about 0.8% to about 5.0%, about 0.9% to about 5.5% by weight, about 0.9% to about 5.0%, about 1.0% to about 5.5% by weight, about 1.0% to about 5.0%, about 1.1% to about 5.5% by weight, about 1.1% to about 5.0%, about 1.2% to about 5.5% by weight, about 1.2% to about 5.0%, about 1.3% to about 5.5% by weight, about 1.3% to about 5.0%, about 1.4% to about 5.5% by weight, or about 1.4% to about 5.0%, by weight.

In a more preferred embodiment in optional combination with any of the above or below aspects or embodiments, *Calendula officinalis* (flower) extract cera is present in the composition in an amount of about 1.5% to about 4.5% by weight such as about 1.5% to about 4.3%, about 1.5% to about 4.0%, about 1.5% to about 3.8%, about 1.7% to about 4.3%, about 1.7% to about 4.0%, about 1.7% to about 3.8%, about 1.9% to about 4.3%, about 1.5% to about 4.0%, about 1.9% to about 3.8%, about 2.1% to about 4.3%, about 2.1% to about 4.0%, about 2.1% to about 3.8%, about 2.3% to about 4.3%, about 2.3% to about 4.0%, or about 2.3% to about 3.8% by weight, even more preferably about 2.5% to about 3.5% by weight. Most preferably, in optional combination with any of the above or below aspects or embodiments, *Calendula officinalis* (flower) extract is present in the composition in an amount of about 3.0% by weight.

In an embodiment in optional combination with any of the above or below aspects or embodiments, the composition comprises about 30% to about 90% by weight of *Olea europaea* (fruit) oil, about 13% to about 53% by weight of *Persea gratissima* oil, about 1% to about 8% by weight of *Euphorbia cerifera* cera and about 0.1% to about 7% by weight of *Calendula officinalis* (flower) extract.

In an embodiment in optional combination with any of the above or below aspects or embodiments, the composition comprises about 40% to about 80% by weight of Olea *europaea* (fruit) oil, about 20% to about 46% by weight of *Persea gratissima* oil, about 2% to about 7% by weight of *Euphorbia cerifera* cera and about 0.5% to about 5.5% by weight of *Calendula officinalis* (flower) extract.

In a more preferred embodiment in optional combination with any of the above or below aspects or embodiments, the composition comprises about 50% to about 70% by weight of *Olea europaea* (fruit) oil, about 27% to about 39% by weight of *Persea gratissima* oil, about 3% to about 6% by weight of *Euphorbia cerifera* cera and about 1.5% to about 4.5% by weight of *Calendula officinalis* (flower) extract.

In a further more preferred embodiment in optional combination with any of the above or below aspects or embodiments, the composition comprises about 58% to about 60% by weight of *Olea europaea* (fruit) oil, about 32% to about 35% by weight of *Persea gratissima* oil, about 4% to about 5% by weight of *Euphorbia cerifera* cera and about 2.5% to about 3.5% by weight of *Calendula officinalis* (flower) extract.

In a most preferred embodiment in optional combination with any of the above or below aspects or embodiments, the composition of the present invention consists of 59% by weight of *Olea europaea* (fruit) oil, 33.5% by weight of *Persea gratissima* oil, 4.5% by weight of *Euphorbia cerifera* cera and 3.0% by weight of *Calendula officinalis* (flower) extract.

In a most preferred embodiment in optional combination with any of the above or below aspects or embodiments, the composition of the present invention consists of 59% by weight of Olea *europaea* fruit oil, 33.5% by weight of *Persea gratissima* oil, 4.5% by weight of *Euphorbia cerifera* cera and 3.0% by weight of *Calendula officinalis* flower extract.

In a further most preferred embodiment in optional combination with any of the above or below aspects or embodiments, the composition of the present invention consists of 59.000% by weight of Olea *europaea* fruit oil, 33.500% by weight of *Persea gratissima* oil, 4.500% by weight of *Euphorbia cerifera* cera and 3.000% by weight of *Calendula officinalis* flower extract.

*Olea europaea* (olive) is a medium-sized tree from the olive tree genus (Olea), which belongs to the olive family (Oleaceae). It is widely cultivated in the Mediterranean area for its fruits. About 90% of the annual harvest of olives is pressed to olive oil, also known as Oleaster oil.

The Olea *europaea* (fruit) oil in the present composition may be obtained by processing the whole fruit including the pulp and kernel. In a preferred embodiment in optional combination with any of the above or below aspects or embodiments, the kernel is separated and only the pulp is used for preparing the olive fruit oil. The (fruit) oil can be produced by grinding olives and extracting the oil by mechanical or chemical means. The olive (fruit) oil in the present invention may be prepared by employing a mechanical process, as is known in the art.

At present, there are two main mechanical processes for extracting oil from olives.

The first process, more conventional and discontinuous, includes a first step of crushing the olives with granite mill stones, a second step of kneading, followed in a third step by the extraction of the oil by means of a pressure, and a subsequent centrifugal separation of the light solids and aqueous phase from the oil.

The second process, more modern, uses continuous mills of various types, such as hammer, blade or disk mill, to crush the olives. The resulting pulpy mass is then stirred in a kneader to cause the fine oil droplets to coalesce. Finally, a centrifuge is used to separate the light solids, the aqueous phase and the oil. Alternatively, the pulpy mass is expeller pressed.

An expeller press is a screw-type machine which presses oil through a caged barrel-like-cavity, using friction and continuous pressure. The screw drives forward to literally squeeze the oil from the compressed seeds. No additional heat is added in this process, but the pressure and friction involved in the pressing process creates heat in the range of 60-99°C.

Preferably, the olive oil (fruit) in the inventive composition may be filtered after centrifugation to eliminate remaining solid particles and to enhance shelf life of the product.

The *Olea europaea* (fruit) oil in the inventive composition may alternatively be obtained commercially, for example from the supplier O&3, product code K0936.

In a preferred embodiment of the present invention in optional combination with any of the above or below aspects or embodiments, the *Olea europaea* fruit oil may be obtained by expeller pressing olive fruits according to processes known to the skilled person. This involves separating the fruits from unwanted components such as chaff or dust and removing the kernel. The olive fruits are then subjected to expeller pressing. The crude oil is filtrated followed by a deodorization process.

In an embodiment of the present invention in optional combination with any of the above or below aspects or embodiments, the inventive composition is formulated without hydrogenated *Olea europaea* (fruit) oil.

*Persea gratissima* (avocado) is a medium-sized, evergreen tree in the laurel family (Lauraceae). Synonyms are *Persea americana, Laurus persea, Persea drymifolia* and *Persea nubigena.* In the United Kingdom, the term "avocado pear" is sometimes used while it is known as "butter fruit" in parts of India and Hong Kong.

The avocado fruit is a single-seeded berry. Inside, in addition to the pulp, it has a large seed that accounts for about 13-18% of the total fruit. Avocado oil can be obtained by mechanically extracting both the seed and the pulp. For preparing the *Persea gratissima* oil in the inventive composition of the present invention, only the fleshy pulp surrounding the avocado seed is pressed. Preferably, the fruits are peeled before pressing.

Avocado oil is usually extracted from the avocado pulp by cold pressing or expeller pressing. Extraction may also be assisted by enzymes or the oil may be hot pressed and extracted using solvents. According to a preferred embodiment of the present invention, the avocado oil in the inventive composition may be extracted from the pulp by expeller pressing to preserve the nutrients contained in the avocado pulp. *Persea gratissima* oil may be obtained according to processes known to the skilled person.

In a preferred embodiment of the present invention in optional combination with any of the above or below aspects or embodiments, the *Persea gratissima* oil may be obtained by expeller pressing avocado pulp according to processes known to the skilled person. This involves separating the fruits from unwanted components such as chaff or dust and then drying the fruits. After removal of the pit, the resulting mass is subjected to expeller pressing. The crude oil is filtrated followed by a refining process.

In a preferred embodiment in optional combination with any of the above or below aspects or embodiments, the inventive composition is formulated without hydrogenated Persea *gratissima* oil.

The *Persea gratissima* oil in the inventive composition may alternatively be obtained commercially, for example from the supplier O&3, product code K0821.

*Euphorbia cerifera* is a species of flowering in the spurge family Euphorbiaceae. It is also known as *Euphorbia antisyphilitica.* To protect against water loss, these plants secrete a thin coating of wax (cera). A common name of *Euphorbia cerifera* cera, which is widely used, is candelilla wax.

The wax is obtained from the leaves and stems of the bush. The plant parts are boiled in water, during which the wax rises to the surface. After skimming, the raw wax is cleaned in several steps. Obtaining the *Euphorbia cerifera* cera (candelilla wax) proceeds by processes known to the skilled person.

In an embodiment of the present invention in optional combination with any of the above or below aspects or embodiments, *Euphorbia cerifera* cera is used in its purified form.

The *Euphorbia cerifera* (candelilla) cera in the inventive composition may alternatively be obtained commercially, for example from KahlWax, product code Kahlwax 2039L.

*Calendula officinalis,* also known as pot marigold, common marigold, ruddles, Mary's gold or Scotch marigold, is a flowering plant in the daisy family Asteraceae. Because the florets of *Calendula officinalis* are edible, they are often used to add color to salads or added to dishes as a garnish and in lieu of saffron. The leaves are edible but are often not palatable. The plant, in particular the flower, is also used to make tea.

In an embodiment of the present invention in optional combination with any of the above or below aspects or embodiments, an extract of the fresh plant material from *Calendula officinalis* may be used. The extraction may be carried out by infusing the plant material in vegetables oils, such as olive oil in a herb:oil ratio from 1:5 to 1:25. During extraction, constant contact between the components may be ensured by continuous stirring. The extraction may be followed by a filtration step.

Typically, the *Calendula officinalis* extract is prepared solely from the flower. In the context of the present invention, the term "flower" includes the receptacle, the sepal(s), the petal(s), the stamina and the pistil(s). In a preferred embodiment of the present invention in optional combination with any of the above or below aspects or embodiments, the *Calendula officinalis* (flower) extract in the inventive composition is an extract of the flower(s) with olive oil. That is, the *Calendula officinalis* (flower) extract is preferably not obtained from extracting the seeds of *Calendula officinalis.* The *Calendula officinalis* (flower) extract may be further preferably formulated without unsaponifiable material. The *Calendula officinalis* (flower) extract used in the composition of the invention may be prepared by methods known to the skilled person.

The *Calendula officinalis* (flower) (extract) in the inventive composition may alternatively be obtained commercially, for example from the supplier O&3, product code K0970.

The composition of the present invention may be applied topically in any suitable form. In a preferred embodiment, the composition is prepared in forms including, but not limited to a balm, a gel, a lotion, a cream, a milk, a salve or ointment. More preferably, the composition is prepared in the form of a balm or lotion, even more preferably, the composition is in the form of a light-weight balm with a creamy texture. Due to its texture, the composition of the present invention can be applied to the skin without the exertion of pressure as it easily spreads and is quickly absorbed into the skin. Further mechanical stress is thus prevented. In an embodiment, the composition of the present invention is applied to the skin by gentle rubbing.

More specifically, the composition of the invention advantageously has a lightweight viscosity that allows it to be distributed on the skin readily without the need to push hard to overcome viscous resistance when applying it to e.g. the nipple. The inventive composition, including the most preferred embodiments thereof described herein, has a viscosity low enough to allow easy spreadability at the site of application, but high enough to prevent immediate dripping away before the composition can be thoroughly spread, or draining away following spreading. This optimal viscosity is especially advantageous for breast-feeding or breastmilk-pumping mothers with sore nipples, since it avoids the need to push hard against viscous resistance in spreading the composition, which otherwise might exacerbate already existing soreness in the nipples and/or nipple region.

The composition may be applied to a body portion to nourish the skin and/or provide relief to dry skin of the body portion.

Dry skin is an uncomfortable condition marked i.a. by scaling and itching. It can occur for a variety of reasons including but not limited to exposure to dry weather conditions, hot water and certain chemicals which can cause the skin to dry out. Dry skin can affect any part of the body such as the hands, elbows, foot, knees and the face.

Dry skin often stems from mechanical stress such as deliberate scratching or from wearing grating or chafing articles of clothing. Specifically breastfeeding mothers often suffer from dry skin, especially of the breast. Sore nipples are also very common because of the baby's suckling, which results in both repeated mechanical stress and repeated cycles of wetting and drying. In an embodiment, the inventive composition is preferably applied to the breast, more preferably to the nipples, most preferably to the nipples of a breastfeeding woman.

Application of the composition of the present invention can be accomplished by any suitable means, such as rubbing, rolling or massaging. To assist in application, it is contemplated that the inventive composition may be applied by any of a variety of known application techniques. For instance, the inventive composition may be held in a pump container, for example in airless pump container, with an application nozzle, so that the inventive composition can be applied as precisely as possible to the intended area. Such application has the advantage of minimizing and contact, and therefore remaining hygienic, while allowing a controlled, metered delivery to the precise area of the body, e.g. the nipple or nipple region, to which application is intended. Accordingly, disclosed herein and contemplated as part of the invention is also a pump container, for instance in airless pump container, containing the composition according to the invention.

Alternatively, the inventive composition can be held in a compressible container comprising an applicator nozzle, for example an angled applicator nozzle, by which the inventive composition can be applied by manually compressing the container to force the composition contained in the container out through a hole in the nozzle, the face of which nozzle can then be used to apply the composition to the precise area of the body, e.g. the nipple or nipple region, to which application is intended. As above in the context of the pump container, application by such a compressible container comprising an applicator nozzle also allows hygienic application to a precise area of the body. Such compressible containers, for example a tube, are well known in the art and art well-suited to convenient transport, allowing relief from e.g. sore nipples and dry skin on the go. Accordingly, disclosed herein and contemplated as part of the invention is also a compressible container, preferably a tube, comprising an applicator nozzle, preferably an angled applicator nozzle, and containing the composition according to the invention.

Alternatively, the inventive composition can be held in a container with a roll-on applicator, the advantages of which mirror the other application methods discussed immediately above, and allows the further advantage of being refillable, provided the roll-on applicator portion of the container is removable. It is thus also contemplated that the composition of the invention could be provided in bulk, waste-reducing package sizes suitable for refilling such containers with roll-on applicators. Accordingly, disclosed herein and contemplated as part of the invention is also a container with a roll-on applicator, preferably a container with a removable roll-on applicator.

### Examples

The present invention will be illustrated by the following non-limiting examples.

### Example 1

A composition is prepared by thoroughly mixing the ingredients in an amount as shown in Table 1.

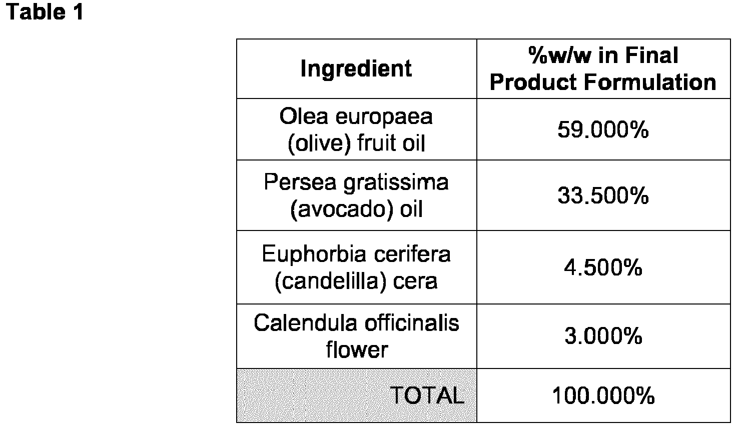

All ingredients are Cosmos Organic Certified.

### Example 2

A composition is prepared by thoroughly mixing the ingredients in an amount as shown in Table 2. The composition is especially suitable for application via a compressible container comprising an applicator nozzle.

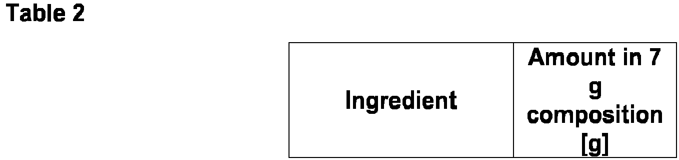

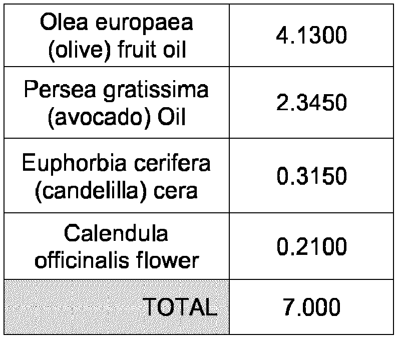

All ingredients are Cosmos Organic Certified.

### Example 3

A composition is prepared by thouroghly mixing the ingredients in an amount as shown in Table 3. The composition is especially suitable for application via an airless pump container with an application nozzle.

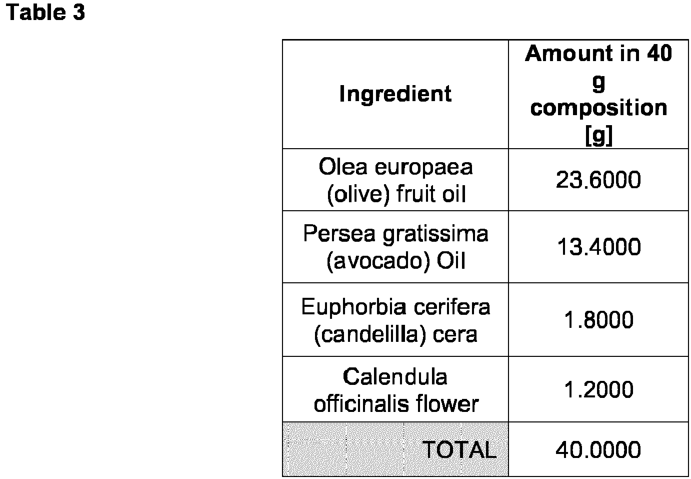

All ingredients are Cosmos Organic Certified.

## Claims

1. A composition comprising *Olea europaea* oil, *Persea gratissima* oil, *Euphorbia cerifera* cera and *Calendula officinalis* extract.

2. The composition according to claim 1, wherein
• the *Olea europaea* oil is *Olea europaea* fruit oil, and/or
• the *Calendula officinalis* extract is *Calendula officinalis* flower extract,
preferably wherein the Olea *europaea* oil is *Olea europaea* fruit oil, and the *Calendula officinalis* extract is *Calendula officinalis* flower extract.

3. The composition according to claim 1 or 2, wherein the composition comprises about 30% to about 90% by weight, preferably about 40% to about 80% by weight, more preferably about 50% to about 70% by weight, even more preferably about 58% to about 60% by weight, most preferably about 59% by weight of the *Olea europaea* oil.

4. The composition according to any one of claims 1-3, wherein the composition comprises about 13% to about 53% by weight, preferably about 20% to about 46% by weight, more preferably about 27% to about 39% by weight, even more preferably about 32% to about 35% by weight, most preferably about 33.5% by weight of the *Persea gratissima* oil.

5. The composition according to any one of claims 1-4, wherein the composition comprises about 1% to about 8% by weight, preferably about 2% to about 7% by weight, more preferably about 3% to about 6% by weight, even more preferably about 4% to about 5% by weight, most preferably about 4.5% by weight of the *Euphorbia cerifera* cera.

6. The composition according to any one of claims 1-5, wherein the composition comprises about 0.1% to about 7% by weight, preferably about 0.5% to about 5.5% by weight, more preferably about 1.5% to about 4.5% by weight, even more preferably 2.5% to about 3.5% by weight, most preferably about 3% by weight of the *Calendula officinalis* extract.

7. The composition according to any one of claims 1-6, wherein the cumulative amount of *Olea europaea* oil and *Persea gratissima* oil in the composition is about 80% to about 98% by weight, preferably about 85% to about 97% by weight, more preferably about 90% to about 95% by weight, most preferably about 92.5% by weight.

8. The composition according to any one of claims 1-7, wherein the cumulative amount of the *Olea europaea* oil, *Persea gratissima* oil, *Euphorbia cerifera* cera and *Calendula officinalis* extract in the composition is more than about 90% by weight, preferably more than about 95% by weight, more preferably more than about 98% by weight.

9. The composition according to claim 8, wherein the composition essentially consists of *Olea europaea* oil, *Persea gratissima* oil, *Euphorbia cerifera* cera and *Calendula officinalis* extract.

10. The composition according to any one of the preceding claims, wherein the ingredients are of certified organic origin.

11. The composition according to anyone of the preceding claims, wherein the composition is in the form of a balm, a cream, a lotion, an ointment, a gel, a milk or a salve..

12. A cosmetic use of the composition according to any one of claims 1-11 for relieving sore nipples and/or dry skin.

13. The use according to claim 12, wherein the composition is administered topically, preferably epicutaneously.
